# EUROPEAN PATENT SPECIFICATION

(11) **EP 0 765 869 B1**
(45) Date of publication and mention of the grant of the patent: **23.08.2000**
(21) Application number: 96202575.5
(22) Date of filing: 16.09.1996
(51) Int. Cl.: C07C 311/19, G03G 9/097

(54) **Monofunctional N-(2-cyanoethenyl) sulfonamides**
Monofunktionelle N-(2-Cyanoethenyl)sulfonamide
N-(2-Cyanoéthényl)sulfonamides monofonctionnels

(30) Priority: 27.09.1995 US 4410; 10.05.1996 US 644758
(43) Date of publication of application: 02.04.1997
(73) Proprietor: EASTMAN KODAK COMPANY, Rochester, New York 14650-2201 (US)
(72) Inventor: Wilson, John C., c/o Eastman Kodak Co., Rochester, New York 14650-2201 (US); Alexandrovich, Peter S., c/o Eastman Kodak Co., Rochester, New York 14650-2201 (US)
(74) Representative: Parent, Yves

(56) References cited:
- US-A- 5 405 727
- AUST. J. CHEM., vol. 46, no. 6, 1993, pages 873-886, XP000196744 H.G. MCFADDEN ET AL:

## Description

The present invention relates to charge control agents which are useful in electrostatographic toner compositions. More particularly, the invention relates to N-(2-cyanoethenyl)sulfonamide charge control agents.

### Cross Reference to Related Applications

The present application is related to copending, commonly assigned United States Serial Number (71743) entitled: TONER COMPOSITIONS CONTAINING N-(2-CYANOETHENYL)SULFONAMIDES. This related application discloses and claims toner compositions using the compounds of the present application as charge control agents in addition to other N-(2-cyanoethenyl)sulfonamides. Reference is made to this related application for data showing the utility of the compounds of the present invention.

### Background of the Invention

In electrography, image charge patterns are formed on a support and are developed by treatment with an electrographic developer containing marking particles which are attracted to the charge patterns. These particles are called toner particles or, collectively, toner. Two major types of developers, dry and liquid, are employed in the development of the charge patterns.

One well-known type of electrostatographic developer comprises a dry mixture of toner particles and carrier particles. Developers of this type are employed in cascade and magnetic brush electro-statographic development processes. The toner particles and carrier paticles differ triboelectrically, such that during mixing to form the developer, the toner particles acquire a charge of one polarity and the carrier particles acquire a charge of the opposite polarity. The opposite charges cause the toner particles to cling to the carrier particles. During development, the electrostatic forces of the latent image, sometimes in combination with an additional applied field, attract the toner particles. The toner particles are pulled away from the carrier particles and become electrostatically attached, in imagewise relation, to the latent image bearing surface. The resultant toner image can then be fixed, by application of heat or other known methods, depending upon the nature of the toner image and the surface, or can be transferred to another surface and then fixed.

Toner particles often include charge control agents, which, desirably, provide high uniform net electrical charge to toner particles without reducing the adhesion of the toner to paper or other medium. Use of charge control agents requires a balancing of shortcomings and desired characteristics to meet a particular situation. Many charge control agents, for example, are colored compounds. Their color makes them less than desirable for use in producing colored images since they interfere with the color rendition of the colorant in the toner composition.

Certain N-(2-cyanoethenyl)sulfonamide compounds are known in the art. The known compounds are all dicyano compounds made by a method that necessarily results in the dicyano compounds. Reference is made to Schulz, et al in Chem. Ber., 100, 2640 (1967). No use is disclosed for the three compounds made in this reference. The compounds have the structures: 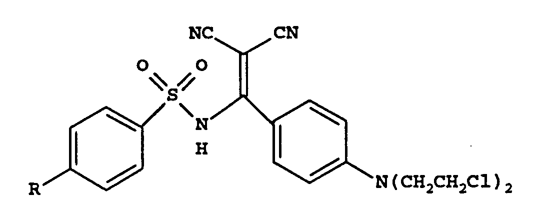 wherein R is hydrogen, CH₃ or NH₂.

There is a continuing need for negative charge control agents.

### Summary of the Invention

The invention provides a charge control agent having the general structure: 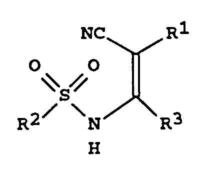 wherein R¹ is selected from the group consisting of hydrogen; alkyl containing from 1 to 20 carbons; cycloalkyl containing from 3 to 18 carbons; unsubstituted aromatic ring systems; aromatic ring systems substituted with one or more alkyl, halo, nitro, cyano, hydroxy, alkoxy, carboxy, carboalkoxy, amino, dialkylamino, acyl, trihalomethyl or alkysulfonyl; and heteroaromatic ring systems; said ring systems having a solitary ring or 2 to 3 linked or fused rings, and containing from 3 to 34 carbons; alkanoyl; alkoxycarbonyl; aminocarbonyl; alkylaminocarbonyl; aralkylaminocarbonyl; alkylsulfonyl; aroyl; aryloxycarbonyl; arylaminocarbonyl; arylsulfonyl; or aroyl, aryloxycarbonyl, arylaminocarbonyl, aralkylaminocarbonyl or arylsulfonyl substituted with one or more alkyl, hydroxy, alkoxy, carboxy, carboalkoxy, nitro, halo, cyano, amino, dialkylamino, acyl, trihalomethyl or alkylsulfonyl; and R² and R³, which can be the same or different, are independently selected from the group consisting of alkyl containing from 1 to 20 carbons; cycloalkyl containing from 3 to 18 carbons; unsubstituted aromatic ring systems; or aromatic ring systems substituted with one or more alkyl, hydroxy, alkoxy, carboxy, carboalkoxy, nitro, halo, cyano, amino, dialkylamino, acyl, trihalomethyl or alkylsulfonyl; heteroaromatic ring systems; said ring systems having a solitary ring or 2 to 3 linked or fused rings, and containing from 3 to 34 carbons; and R³ may also be ethenyl, unsubstituted or substituted with alkyl containing from 1 to 20 carbons or aryl containing from 5 to 10 carbons or aryl substituted with alkyl, hydroxy, carboxy, carboalkoxy, nitro, halo, cyano, amino, dialkylamino, acyl, trihalomethyl, or alkylsulfonyl, wherein R¹ cannot be carboethoxy when R² is phenyl and R³ is methyl.

### Decription of Preferred Embodiments

The term "charge control," as used herein, refers to a propensity of a toner addendum to modify the triboelectric charging properties of the resulting toner.

The compounds of the present invention, are sulfonamides and have the general structure: 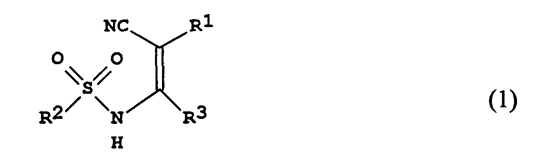 wherein the various substituents are as described above. It is noted that in comparison to the definition in the above identified related application, R¹ here can not be cyano. Thus, the compounds of the Schulz, et al cited above are excluded from the compounds of the present invention. In addition, the compounds of the present invention, not being dicyano compounds, can not be made using the method of Schulz, et al.

It is to be understood that the general structure (1) set forth above includes both geometrical isomers: 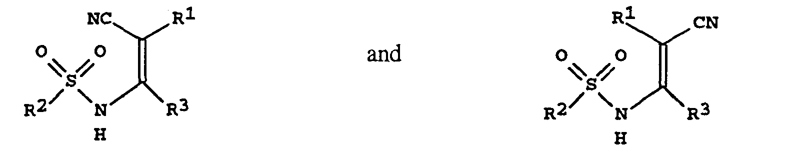 where R¹, R² and R³ are as defined above.

It is also to be understood that the sulfonamides of the invention can tautomerize. Thus, structure (1) could, in many cases, also be represented:

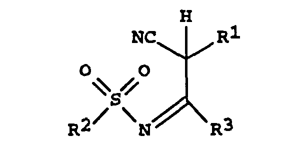

For the sake of brevity, alternate geometric isomeric and tautomeric forms will not be illustrated herein. However, structural formulas should be understood to be inclusive of these alternate forms.

The charge control agents of the invention also are essentially colorless and exhibit excellent thermal stability in air.

It is preferred that one and preferrably both of R² and R³ be aromatic. Where R² and R³ are alkyl, the charging rate is less than desired. Thus, embodiments of the sulfonamides of the invention which are currently preferred, can be represented by the general structure: 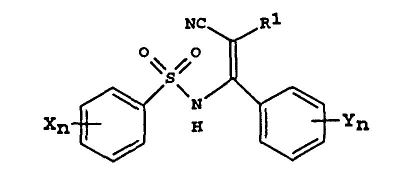 wherein X and Y, each of which can be the same or different, are hydrogen, alkyl, hydroxy, alkoxy, carboxy, carboalkoxy, halo, nitro, cyano, amino, dialkylamino, acyl, trihalomethyl or alkylsulfonyl and R¹ is as defined above; and each n is independently an integer of from 0 to 5.

The currently most preferred embodiments of the sulfonamides of the invention can be represented by the general structure: 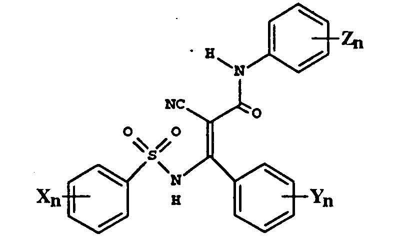 wherein X and Y are as defined above and Z, each of which can be the same or different, is hydrogen, alkyl, hydroxy, alkoxy, carboxy, carboalkoxy, halo, nitro, cyano, amino, dialkylamino, acyl, trihalomethyl or alkylsulfonyl; and each n is independently an integer of from 0 to 5.

Specific examples of sulfonamide charge control agents of the invention are:

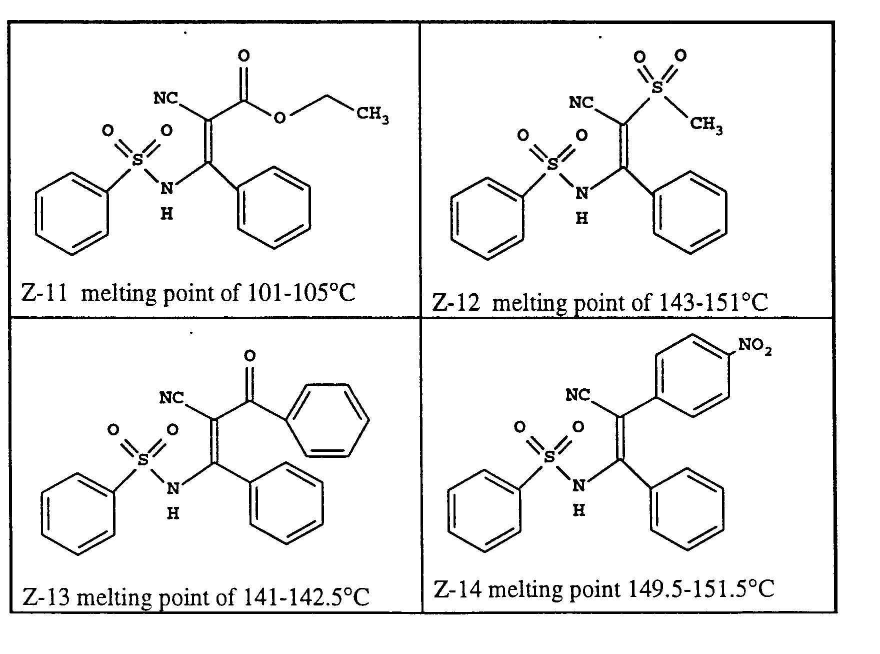

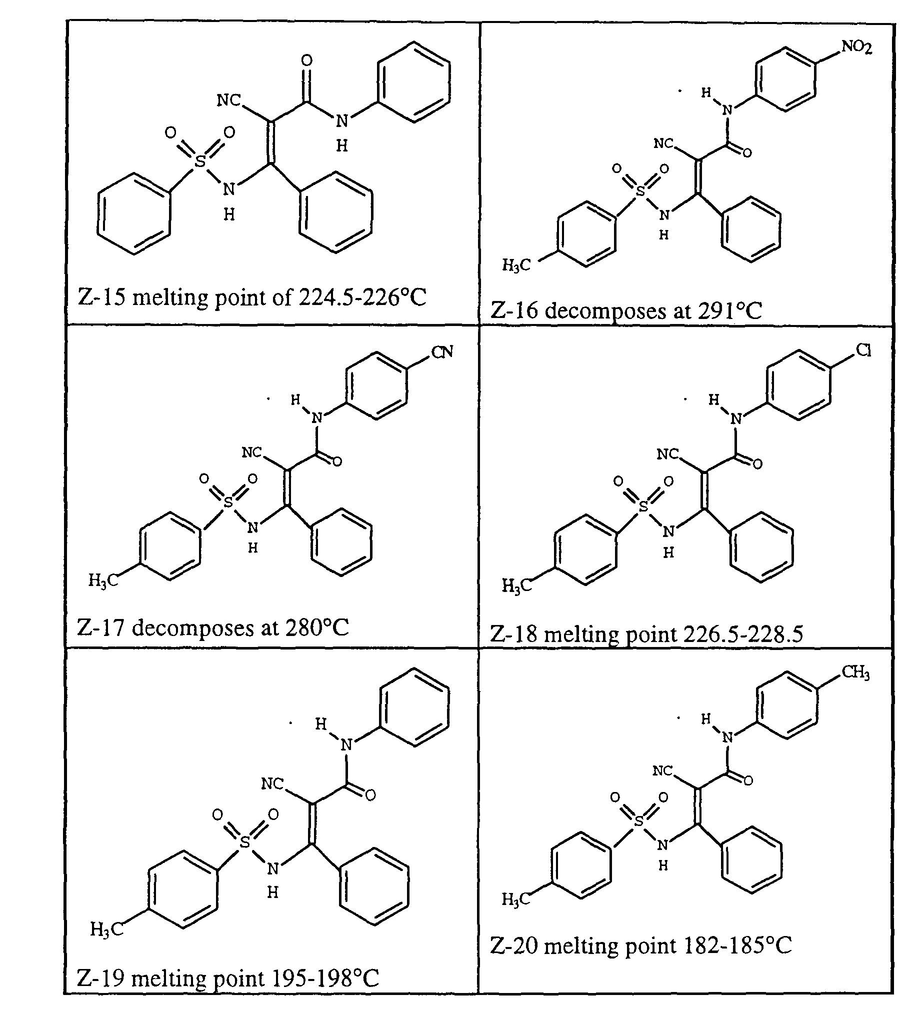

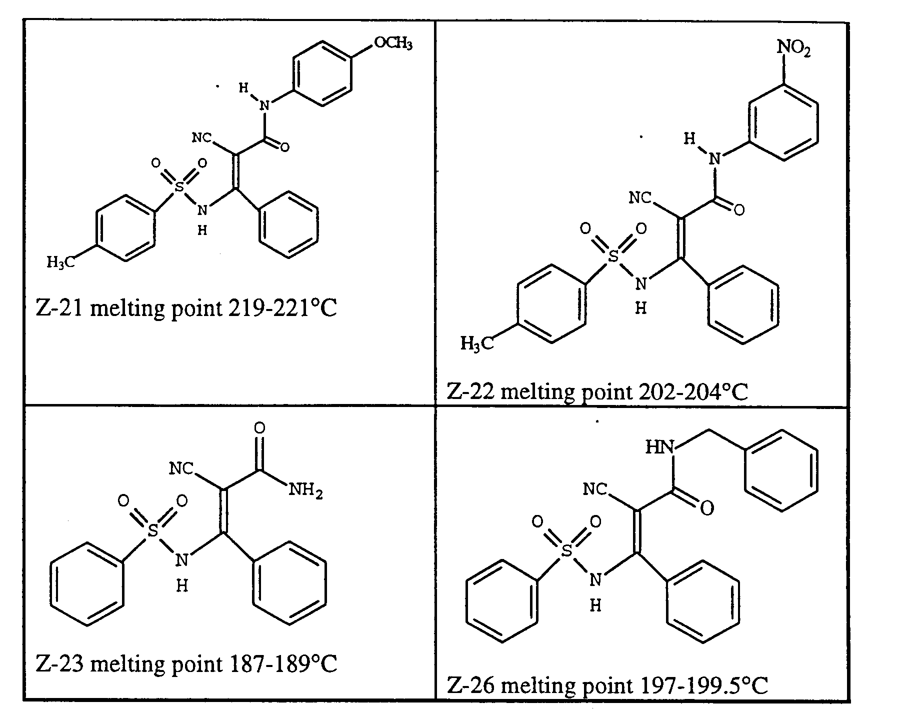

The currently preferred compounds are Z-16, Z-17, Z-18, Z-21 and X-22. These compounds, within the preferred structure, have excellent combinations of properties.

The sulfonamides useful in the invention can be prepared in accordance with the following reaction scheme:

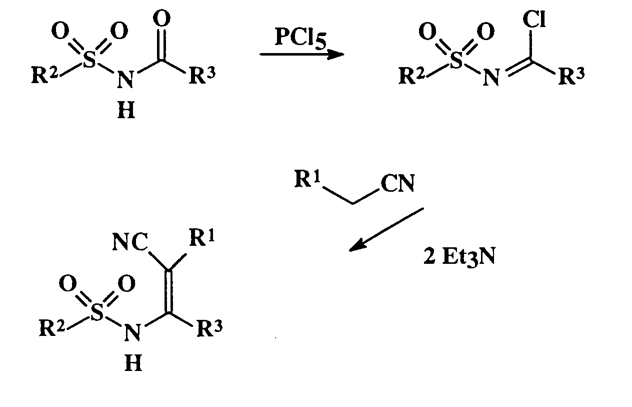

The first reaction is generally known in the art and is described for example in Barnikow and Richter, [Z, Chem., 20(3), 97(1980)]; the second reaction is known in the context of saccharin chemistry but has not been applied to compounds similar to the present invention. Reference is made to Melchiorre, et al; Ann. Chim. (Rome) 1971, 61(6), 399.

The invention is further illustrated by the following Examples. N-acylsulfonamides were prepared by the method disclosed by Kemp and Stephen, J. Chem. Soc., 1948, 11. N-sulfonylcarboximidoyl chlorides were prepared by the method disclosed by Barnikow and Richter, Z. Chem., 20(3), 97 (1980). 2-Cyanoacetamides were prepared by the method disclosed in Ried and Schleimer, Ann., 626, 98 (1959). All other chemicals were commercially available. All melting points for the compounds described above are uncorrected. Thermal stabilities (TGA) in air were determined with a Perkin-Elmer Series 7 Thermal Analysis System at a heating rate of 10°C/min from 25-500°C. Elemental analyses were performed by combustion techniques. NMR, IR and elemental analysis confirmed the proposed structure in each case.

### Example

### Preparation of Charge control Agent

A representative synthesis for charge control agent Z-19 is given below. All of the other charge control agent examples given above were prepared in an analogous manner using the appropriate starting materials.

A sulfonamide charge control agent having the structural formula: 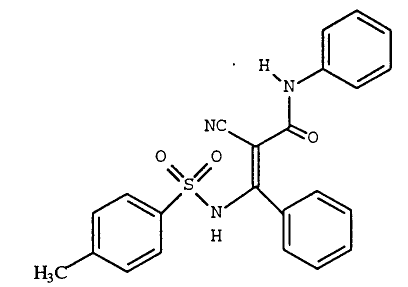 was prepared as follows:

To a mixture of 8.01 g (50 mmol) of 2-cyanoacetanilide, 14.69 g (50 mmol) of N-(4-methylphenylsulfonyl)benzenecarboximidoyl chloride and 200 mL of methylene chloride was added 10.12 g (100 mmol) of triethylamine over 10 minutes followed by 25 mL of methylene chloride rinse. The reaction mixture was stirred for another 1 hr; washed three times with 10% HCl and once with water, dried over magnesium sulfate and concentrated. The solid residue was recrystallized from acetonitrile, collected and dried. The yield of product was 14.64 g (70.1% of theory); mp = 195-198°C.

| | | | | |
|---|---|---|---|---|
| Elem. analysis for C₂₃H₁₉N₃ O₃S | C, 66.17; | H, 4.59; | N, 10.06; | S, 7.68 |
| Found | C, 66.16; | H, 4.75; | N, 10.03, | S, 7.33 |

The compound was stable in air to 268°C.

While specific embodiments of the invention have been shown and described herein for purposes of illustration, the protection afforded by any patent which may issue upon this application is not strictly limited to a disclosed embodiment; but rather extends to modifications and arrangements which fall fairly within the scope of the claims which are appended hereto.

## Claims

1. A compound having the general structure: 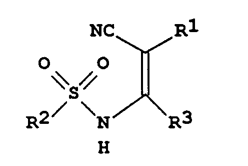 wherein R¹ is selected from the group consisting of hydrogen; alkyl containing from 1 to 20 carbons; cycloalkyl containing from 3 to 18 carbons;
unsubstituted aromatic ring systems; aromatic ring systems substituted with one or more alkyl, halo, nitro, cyano, hydroxy, alkoxy, carboxy, carboalkoxy, amino, dialkylamino, acyl, trihalomethyl or alkysulfonyl; and heteroaromatic ring systems; said ring systems having a solitary ring or 2 to 3 linked or fused rings, and containing from 3 to 34 carbons; alkanoyl; alkoxycarbonyl; aminocarbonyl; alkylaminocarbonyl; aralkylaminocarbonyl; alkylsulfonyl; aroyl; aryloxycarbonyl; arylaminocarbonyl; arylsulfonyl; or aroyl, aryloxycarbonyl, arylaminocarbonyl, aralkylaminocarbonyl or arylsulfonyl substituted with one or more alkyl, hydroxy, alkoxy, carboxy, carboalkoxy, nitro, halo, cyano, amino, dialkylamino, acyl, trihalomethyl or alkylsulfonyl; and R² and R³, which can be the same or different, are independently selected from the group consisting of alkyl containing from 1 to 20 carbons; cycloalkyl containing from 3 to 18 carbons; unsubstituted aromatic ring systems; or aromatic ring systems substituted with one or more alkyl, hydroxy, alkoxy, carboxy, carboalkoxy, nitro, halo, cyano, amino, dialkylamino, acyl, trihalomethyl or alkylsulfonyl; heteroaromatic ring systems; said ring systems having a solitary ring or 2 to 3 linked or fused rings, and containing from 3 to 34 carbons; and R³ may also be ethenyl, unsubstituted or substituted with alkyl containing from 1 to 20 carbons or aryl containing from 5 to 10 carbons or aryl substituted with alkyl, hydroxy, carboxy, carboalkoxy, nitro, halo, cyano, amino, dialkylamino, acyl, trihalomethyl, or alkylsulfonyl, wherein R¹ cannot be carboethoxy when R² is phenyl and R³ is methyl.

2. A compound according to claim 1 having the general structure: 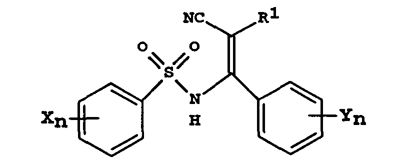 wherein X and Y, each of which can be the same or different, are hydrogen, alkyl, hydroxy, alkoxy, carboxy, carboalkoxy, halo, nitro, cyano, amino, dialkylamino, acyl, trihalomethyl or alkylsulfonyl; and each n is independently an integer of from 0 to 5.

3. A compound according to claim 1 having the general structure: 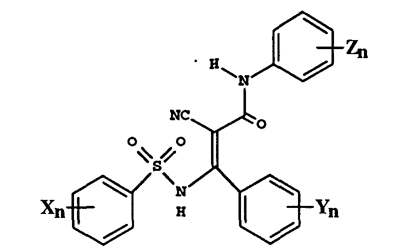 wherein X and Y, each of which can be the same or different, are hydrogen, alkyl, hydroxy, alkoxy, carboxy, carboalkoxy, halo, nitro, cyano, amino, dialkylamino, acyl, trihalomethyl or alkylsulfonyl; Z, each of which can be the same or different, is hydrogen, alkyl, hydroxy, alkoxy, carboxy, carboalkoxy, halo, nitro, cyano, amino, dialkylamino, acyl, trihalomethyl or alkylsulfonyl; and each n is independently an integer of from 0 to 5.

4. A compound according to claim 3 having the formula:

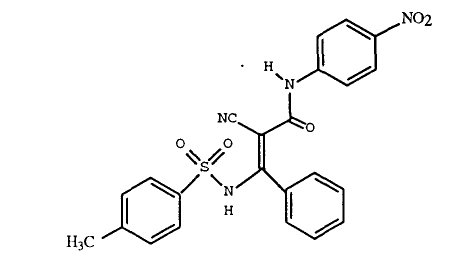

5. A compound according to claim 3 having the formula:

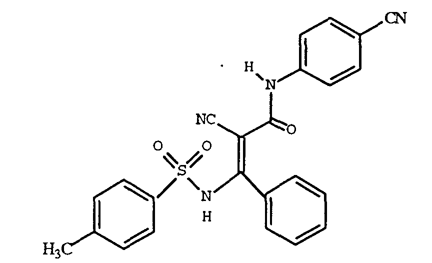

6. A compound according to claim 3 having the formula:

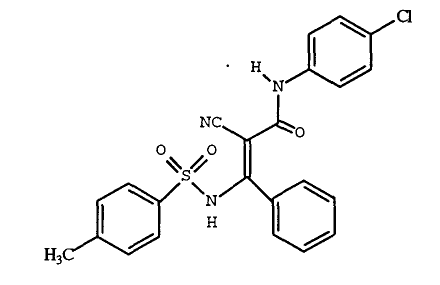

7. A compound according to claim 3 having the formula:

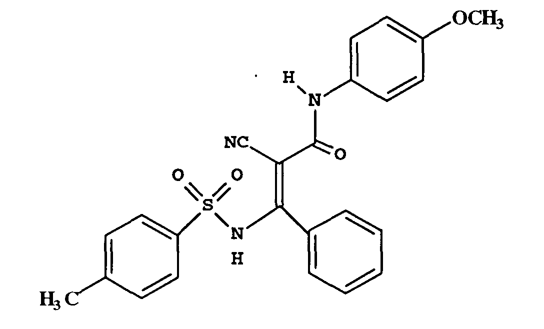

8. A compound according to claim 3 having the formula:

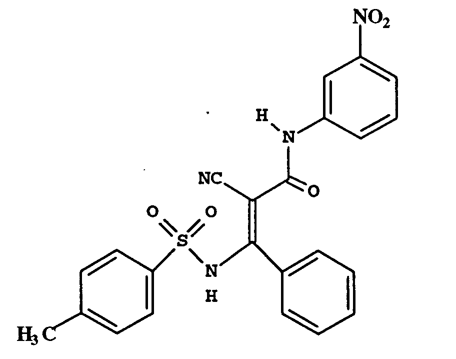

## Patentansprüche

1. Verbindung der allgemeinen Strukturformel: 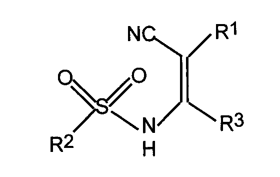 **dadurch gekennzeichnet**, dass R¹ aus der Gruppe Wasserstoff auswählbar ist, Alkyl 1 bis 20 Kohlenstoffatome enthält, Cycloalkyl 3 bis 18 Kohlenstoffatome, unsubstituierte aromatische Ringsysteme, aromatische Ringsysteme mit einem oder mehreren Alkyl-, Halogen-, Nitro-, Cyano-, Hydroxy-, Alkoxy-, Carboxy-, Carboalkoxy-, Amino-, Dialkylamino-, Acyl-, Trihalogenmethyl- oder Alkysulfonyl-substituenten; und heteroaromatische Ringsysteme, wobei die Ringsysteme einen Einzelring oder 2 bis 3 miteinander verbundene oder kondensierte Ringe aufweisen und 3 bis 34 Kohlenstoffatome enthalten; Alkanoyl; Alkoxycarbonyl; Aminocarbonyl; Alkylaminocarbonyl; Aralkylaminocarbonyl; Alkylsulfonyl; Aroyl; Aryloxycarbonyl; Arylaminocarbonyl; Arylsulfonyl; oder Aroyl, Aryloxycarbonyl, Arylaminocarbonyl; Aralkylaminocarbonyl oder Arylsulfonyl mit einem oder mehreren Alkyl-, Hydroxy-, Alkoxy- Carboxy-, Carboalkoxy-, Nitro-, Halogen-, Cyano-, Amino-, Dialkylamino-, Acyl-, Trihalogenmethyl- oder Alkylsulfonyl-substituenten; wobei R² und R³, die gleich oder unterschiedlich sein können, unabhängig voneinander aus der Gruppe auswählbar sind, die aus Alkyl mit 1 bis 20 Kohlenstoffatomen; Cycloalkyl mit 3 bis 18 Kohlenstoffatomen, unsubstituierten aromatischen Ringsystemen oder aromatischen Ringsystemen mit einem oder mehreren Alkyl-, Hydroxy-, Alkoxy- Carboxy-, Carboalkoxy-, Nitro-, Halogen-, Cyano-, Amino-, Dialkylamino-, Acyl-, Trihalogenmethyl- oder Alkylsulfonylsubstituenten; heteroaromatischen Ringsystemen, die einen Einzelring oder 2 bis 3 miteinander verbundene oder kondensierte Ringe aufweisen und 3 bis 34 Kohlenstoffatome enthalten, besteht; wobei R³ auch Ethenyl sein kann, unsubstituiert oder substituiert mit 1 bis 20 Kohlenstoffatome aufweisendem Alkyl oder 5 bis 10 Kohlenstoffatome enthaltendem Aryl, oder Aryl, das mit Alkyl, Hydroxy, Carboxy, Carboalkoxy, Nitro, Halogen, Cyano, Amino, Dialkylamino, Acyl, Trihalogenmethyl oder Alkylsulfonyl substituiert ist, und wobei R¹ nicht Carboethoxy sein kann, wenn R² Phenyl und R³ Methyl ist.

2. Verbindung nach Anspruch 1 der allgemeinen Struktur: 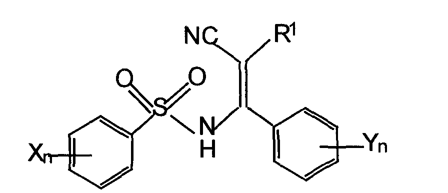 dadurch gekennzeichnet, dass X und Y, die entweder gleich oder unterschiedlich sein können, Wasserstoff, Alkyl, Hydroxy, Alkoxy, Carboxy, Carboalkoxy, Halogen, Nitro, Cyano, Amino, Dialkylamino, Acyl, Trihalogenmethyl oder Alkylsulfonyl sind und dass beide n, unabhängig voneinander, eine ganze Zahl zwischen 0 und 5 sind.

3. Verbindung nach Anspruch 1 der allgemeinen Struktur: 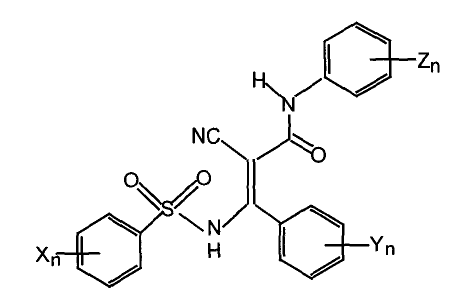 dadurch gekennzeichnet, dass X und Y, die entweder gleich oder unterschiedlich sein können, Wasserstoff, Alkyl, Hydroxy, Alkoxy, Carboxy, Carboalkoxy, Halogen, Nitro, Cyano, Amino, Dialkylamino, Acyl, Trihalogenmethyl oder Alkylsulfonyl sind; dass alle Z gleich oder unterschiedlich sind und Wasserstoff, Alkyl, Hydroxy, Alkoxy, Carboxy, Carboalkoxy, Halogen, Nitro, Cyano, Amino, Dialkylamino, Acyl, Trihalogenmethyl oder Alkylsulfonyl sind; und dass jedes n unabhängig von den anderen eine ganze Zahl zwischen 0 und 5 ist.

4. Verbindung nach Anspruch 3 der Formel:

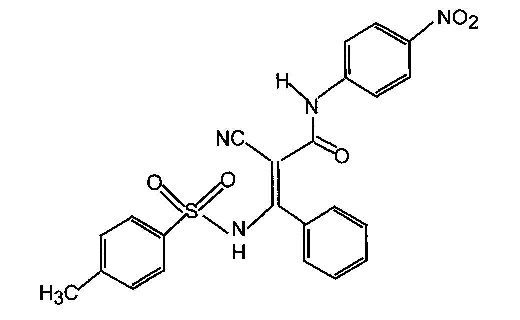

5. Verbindung nach Anspruch 3 der Formel:

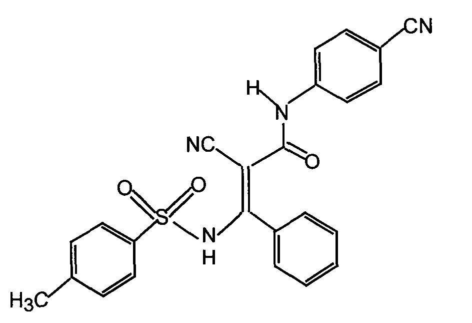

6. Verbindung nach Anspruch 3 der Formel:

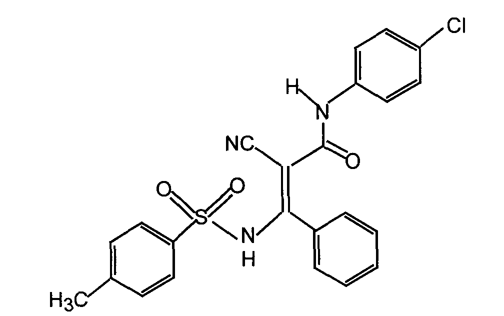

7. Verbindung nach Anspruch 3 der Formel:

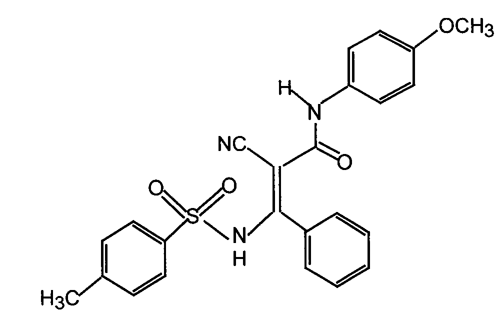

8. Verbindung nach Anspruch 3 der Formel:

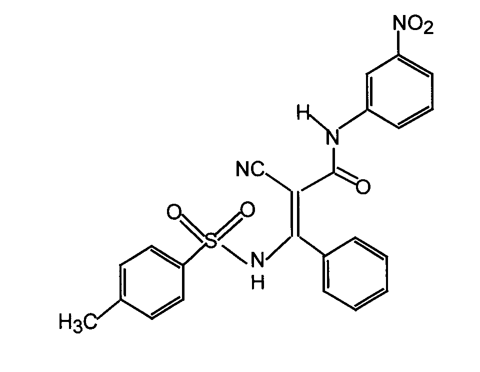

## Revendications

1. Composé représenté par la structure générale : 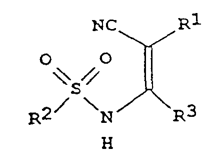 où R¹ est choisi parmi le groupe comprenant l'hydrogène, un groupe alkyle de 1 à 20 atomes de carbone, cycloalkyle de 3 à 18 atomes de carbone, des systèmes cycliques aromatiques non substitués, des systèmes cycliques aromatiques substitués par un ou plusieurs groupes alkyle, halo, nitro, cyano, hydroxy, alcoxy, carboxy, carboalcoxy, amino, dialkylamino, acyle, trihalométhyle ou alkylsulfonyle et des systèmes cycliques hétéroaromatiques ; lesdits systèmes cycliques comprenant un cycle simple vou 2 ou 3 cycles liés ou condensés de 3 à 34 atomes de carbone, des groupes alcanoyle, alcoxycarbonyle, aminocarbonyle, alkylaminocarbonyle, aralkylaminocarbonyle, alkylsulfonyle, aroyle, aryloxycarbonyle, arylaminocarbonyle, arylsulfonyle ou aroyle, aryloxycarbonyle, arylaminocarbonyle, arakylaminocarbonyle ou arylsulfonyle substitués par un ou plusieurs groupes alkyle, hydroxy, alcoxy, carboxy, carboalcoxy, nitro, halo, cyano, amino, dialkylamino, acyle, trihalométhyle ou alkylsulfonyle ; et R² et R³, qui peuvent être identiques ou différents, sont indépendamment choisis parmi le groupe comprenant les groupes alkyle de 1 à 20 atomes de carbone, cycloalkyle de 3 à 18 atomes de carbone, des systèmes cycliques aromatiques non substitués ou des systèmes cycliques aromatiques substitués par un ou plusieurs groupes alkyle, hydroxy, alcoxy, carboxy, carboalcoxy, nitro, halo, cyano, amino, dialkylamino, acyle, trihalométhyle ou alkylsulfonyle et des systèmes cycliques hétéroaromatiques, lesdits systèmes cycliques comprenant un cycle simple ou 2 ou 3 cycles liés ou condensés de 3 à 34 atomes de carbone et R³ peut également être un groupe éthényle substitué ou non par un groupe alkyle de 1 à 20 atomes de carbone ou un groupe aryle de 5 à 10 atomes de carbone ou un groupe aryle substitué par des groupes alkyle, hydroxy, carboxy, carboalcoxy, nitro, halo, cyano, amino, dialkylamino, acyle, trihalométhyle ou alkylsulfonyle, où R¹ ne peut être un groupe carboéthoxy lorsque R² est un groupe phényle et R³ est un groupe méthyle.

2. Composé selon la revendication 1 représenté par la structure générale : 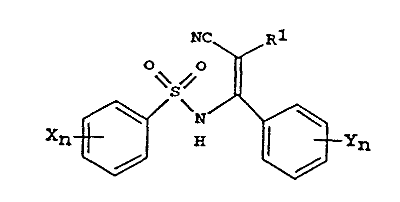 où X et Y, chacun d'entre eux pouvant être différent ou identique, sont l'hydrogène, un groupe alkyle, hydroxy, alcoxy, carboxy, carboalcoxy, halo, nitro, cyano, amino, dialkylamino, acyle, trihalométhyle ou alkylsulfonyle et chaque n est indépendamment un entier de 0 à 5.

3. Composé selon la revendication 1 représenté par la structure générale : 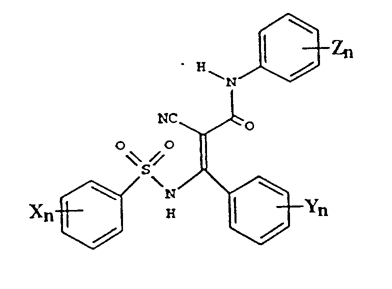 où X et Y, chacun d'entre eux pouvant être différent ou identique, sont l'hydrogène, un groupe alkyle, hydroxy, alcoxy, carboxy, carboalcoxy, halo, nitro, cyano, amino, dialkylamino, acyle, trihalométhyle ou alkylsulfonyle ; les groupes Z, chacun d'entre eux pouvant être différent ou identique, sont l'hydrogène, un groupe alkyle, hydroxy, alcoxy, carboxy, carboalcoxy, halo, nitro, cyano, amino, dialkylamino, acyle, trihalométhyle ou alkylsulfonyle et chaque n est indépendamment un entier de 0 à 5.

4. Composé selon la revendication 3 représenté par la formule :

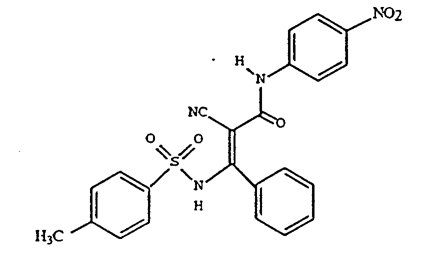

5. Composé selon la revendication 3 représenté par la formule :

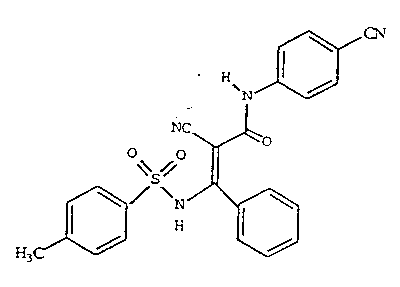

6. Composé selon la revendication 3 représenté par la formule :

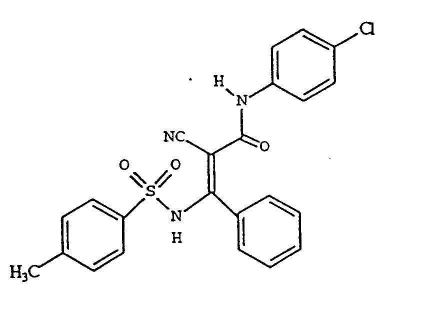

7. Composé selon la revendication 3 représenté par la formule :

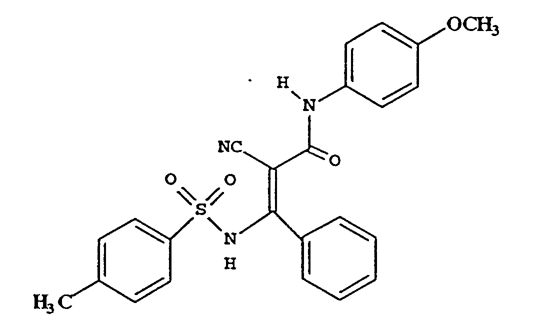

8. Composé selon la revendication 3 représenté par la formule :
